# EUROPEAN PATENT SPECIFICATION

(11) **EP 1 129 664 B1**
(45) Date of publication and mention of the grant of the patent: **26.07.2006**
(21) Application number: 01200760.5
(22) Date of filing: 01.03.2001
(51) Int. Cl.: A61B 6/04

(54) **Table for X-ray examination**
Röntgenuntersuchungstafel
Table pour examen à rayons X

(30) Priority: 03.03.2000 NL 1014555
(43) Date of publication of application: 05.09.2001
(73) Proprietor: L. KONINGS BEHEER B.V., 6071 XT Swalmen (NL)
(72) Inventor: van Son, Josef Johannes Maria, 6071 XT Swalmen (NL)
(74) Representative: Vollebregt, Cornelis Jacobus

(56) References cited:
- WO-A-96/37088
- US-A- 4 602 378
- US-A- 5 764 724
- US-A- 6 027 247

## Description

The invention relates to a table for X-ray examination, comprising a tabletop supported by a frame, on which a person to be examined can lie in a horizontal position of the tabletop, whilst an image recording element, is connected to the tabletop, which image recording element is movable in a direction substantially parallel to the longitudinal direction of the tabletop and adjustable between a position in which the image recording element extends approximately perpendicular to the imaginary plane comprising the upperside of the tabletop and a position in which the image recording element extends substantially parallel to said imaginary plane.

Such a device is known from US-A-5,764,724. By the device according to US-A-5,764,724 the image recording element is movable into several different positions. However, in a position in which the image recording element extends approximately perpendicular to the imaginary plane comprising the upperside of the tabletop, the distance between the image recording element and he upperside of the tabletop is fixed. This renders the device not suitable for taking a series of images on different positions in said direction.

It is an object of the invention to overcome the disadvantages of this prior art device.

This object is being accomplished in that the image recording element is supported by an arm which is pivotably about a pivot that extends parallel to the tabletop, along which arm the image recording element is movable and adjustable in several positions.

Because the image recording element is movable and adjustable in several positions along said arm, images on different positions in said direction can be taken.

By using the construction according to the invention, a device of simple design having a wide range of applications is obtained. By means of a single image recording element it is possible not only to obtain X-ray images both of a person in standing position and of a person in reclined position, but also, in the horizontal position of the image recording element, to produce X-ray images of parts of the body being placed on the image recording element, such as a hand, for example.

A further extension of the range of applications of the table is obtained in that the image recording element is movable from one side of the tabletop to the other, in a direction transversely to the longitudinal axis of the tabletop.

The invention will now be explained in more detail by means of an embodiment of a table according to the invention as shown in the accompanying figures.
Figure 1 is a view, partially in side elevation and partially in section, of a table according to the invention, wherein the tabletop is shown in a vertical position thereof.
Figure 2 is a side view of Figure 1.
Figure 3 is a side elevation substantially corresponding to Figure 2, wherein the image recording element is represented in various adjusted positions thereof.
Figure 4 is a view, partially in section and partially in front elevation, of the table according to the invention, wherein the tabletop is shown in a horizontal position thereof.
Figures 5 and 6 are larger-scale cross-sectional views of Figure 4, wherein the image recording element is shown in various positions thereof.
Figures 7 and 8 show further possible adjusted positions of the image recording element.

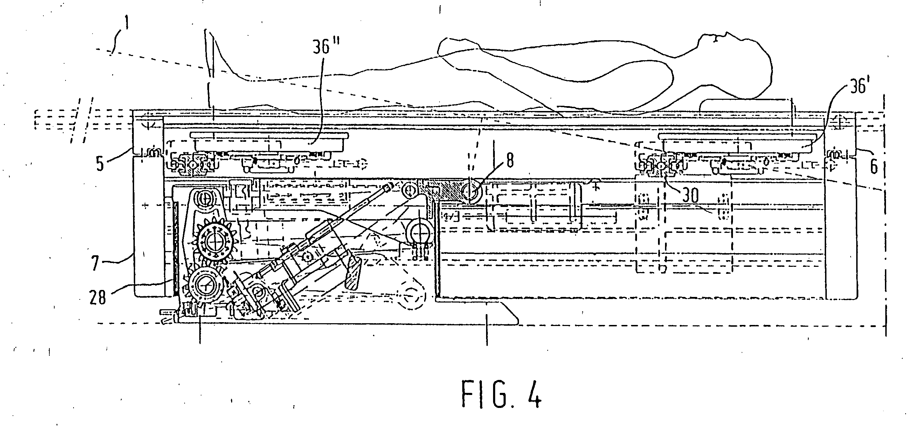

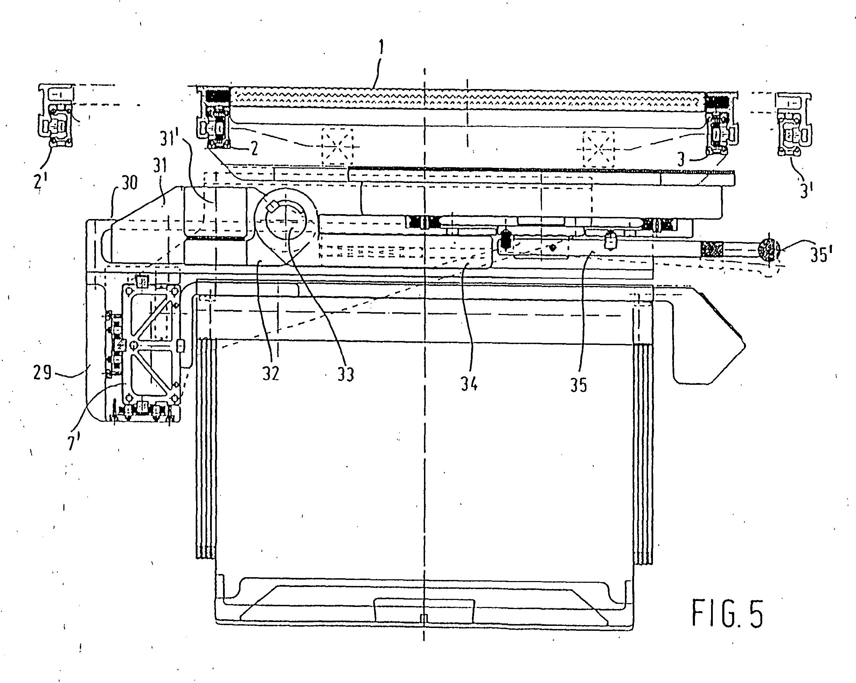

The device which is shown in the figures comprises a tabletop 1 which transmits X-rays, which tabletop is supported by two beams 2, 3 extending in the longitudinal direction of the tabletop, which are disposed near the sides of the tabletop (Figure 5), along which beams the tabletop 1 is movable in longitudinal direction, that is, parallel to its longitudinal axis, to the left and to the right in Figure 4, as is indicated by means of dotted lines drawn in line with tabletop 1 in Figure 4.

The ends of the frame beams 2 and 3 are connected to cross beams 5, 6, which are disposed near the ends of the tabletop, seen in Figure 4, which cross beams are movable in a direction transversely to the longitudinal direction of the tabletop with respect to a frame member 7 supporting the tabletop, as is indicated with position 2' of beam 2 and position 3' of beam 3 in Figure 5.

Frame member 7 is connected to one end of a support 9 by means of a pivot pin 8 which extends horizontally and transversely to the longitudinal direction of tabletop 1.

Connected to support 9, by means of pivots 10 and 11 disposed near pivot pin 8 and extending parallel thereto, are the ends of two arms 12 and 13, with arm 13 only being indicated by means of a chain-dotted line. The ends of the arms 12 and 13 remote from pivots 10 and 11 are connected to a connecting piece 16 by means of pivots 14 and 15, respectively, extending parallel to pivots 10 and 11, all this in such a manner that arms 12 and 13 and the associated pivots 10, 11 and 14, 15 form a parallelogram construction.

Furthermore connected to connecting piece 16 are the ends of two arms 17 and 18, with arm 17 being pivotally connected to connecting piece 16 by means of pivot 14 and arm 18 being connected to connecting piece 16 by means of a pivot 19 which extends parallel to pivot 14. Like arm 13, arm 17 is indicated by means of chain-dotted lines.

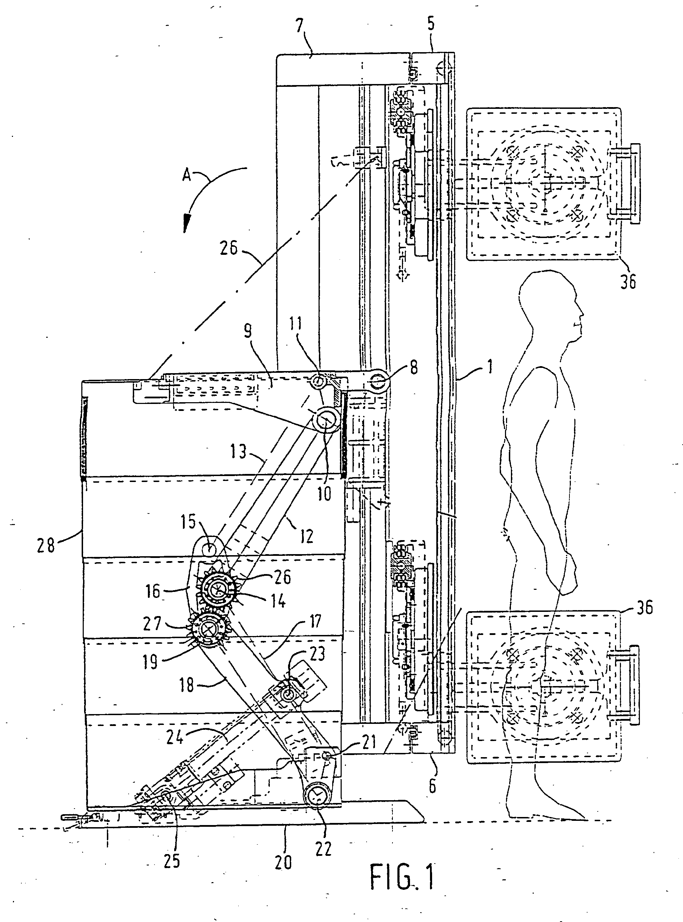

In Figure 1, arms 17 and 18 extend downwards from connecting piece 16 in a direction away from arms 12 and 13, which extend upwards from connecting piece 16.

The lower ends of arms 17 and 18, seen in Figure 1, are pivotally connected, by means of two parallel pivots 21 and 22, to a frame 20 of the device, which is disposed on the floor, all this in such a manner that also the arms 17 and 18 and the associated pivots 14, 19, 21 and 22 form a parallelogram construction.

Connected to arm 18, by means of a pivot 23 extending parallel to pivots 22 and 19, is the end of a setting cylinder 24. The other end of setting cylinder 24 is connected to frame 20 by means of a pivot 25 extending parallel to pivot 23.

As is furthermore shown in Figure 1, the arms 18 and 12 are interconnected by means of a gears 26 and 27, respectively, which are mounted on pivots 14 and 19.

As is furthermore illustrated in a chain-dotted line 26 in Figure 1, an adjusting element 26, for example a setting cylinder or the like, is mounted between the frame member 7 supporting tabletop 1 and support 9. After being unlocked, frame member 7 can be pivoted from the position which is shown in Figure 1, in which the tabletop 1 occupies a vertical position and is locked in position for use of the device, through an angle of ± 90° about pivot 8, as is indicated by arrow A, by means of said adjusting element 26 in order to move the tabletop 1 to a horizontal position. The tabletop that has been moved to a horizontal position can be moved upwards and downwards by means of setting cylinder 24, by pivoting of the above-described, interconnected parallelogram mechanisms, with a lowermost position of tabletop 1 being illustrated in Figure 4. In this position a person to be examined can easily position himself on the tabletop, whilst the tabletop can be moved upwards in its horizontal position in order to bring the person within easier reach of the operating staff. The tabletop can also be disposed in a slightly inclined position by pivoting about pivot 8, as is indicated by means of dotted line 1' in Figure 4.

As will furthermore be apparent from Figures 1 and 4, the above-described mechanism for moving the tabletop upwards and downwards can be screened by a number of tubular members 28, which can telescope with respect to each other.

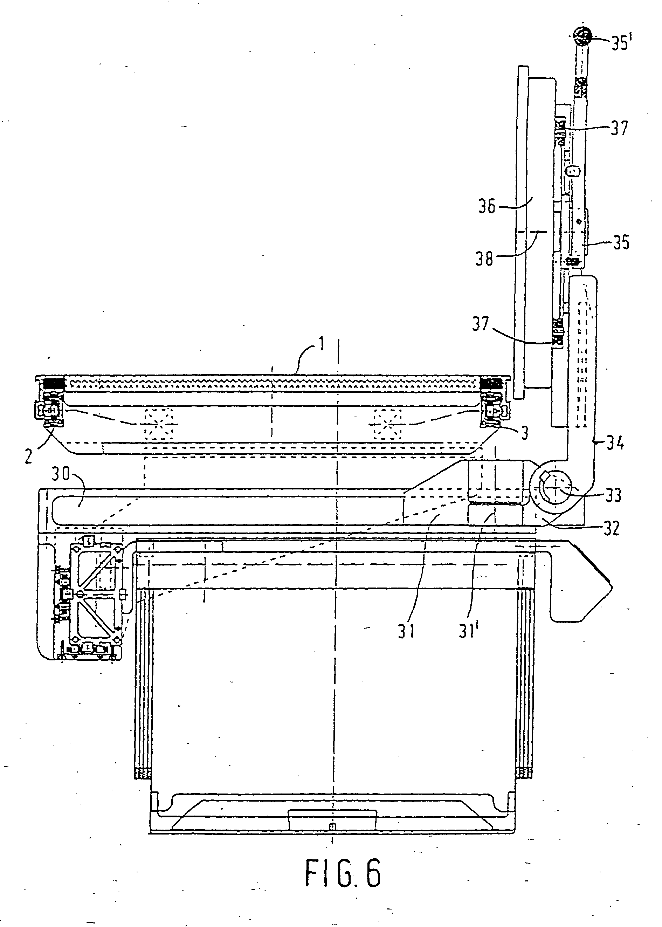

Frame member 7 comprises a frame beam 7' extending in the longitudinal direction of the tabletop, along which a sliding member or trolley 29 (Figure 5) is movable, for example by means of a chain drive. Connected to sliding member 29 is a support or I-beam 30 extending under the tabletop, transversely to the longitudinal direction thereof. A sliding member 31 is movable along said support 30. A connecting piece 32 is pivotally connected to sliding member 31 by means of a pivot 31' extending vertically (seen in Figures 5 and 6) and thus perpendicularly to the tabletop 1. An arm 34 is pivotally connected to said connecting piece by means of a pivot 33 extending parallel to tabletop 1. A supporting member 35 is movable in the longitudinal direction of arm 34 with respect to said arm 34 by means of a grip 35' and capable of being locked in several desired positions with respect to arm 34.

Supporting member 35 supports an image recording element 36 by means of wheels 37, all this in such a manner that the image recording element 36 is pivotable with respect to supporting member 35 and capable of being adjusted about a pivot 38 that crosses pivot 33 perpendicularly.

In the illustrated embodiment, image recording element 36 is a flat, box-like member, which houses sensors capable of detecting X-rays, which sensors transmit signals to the viewing screen or the like.

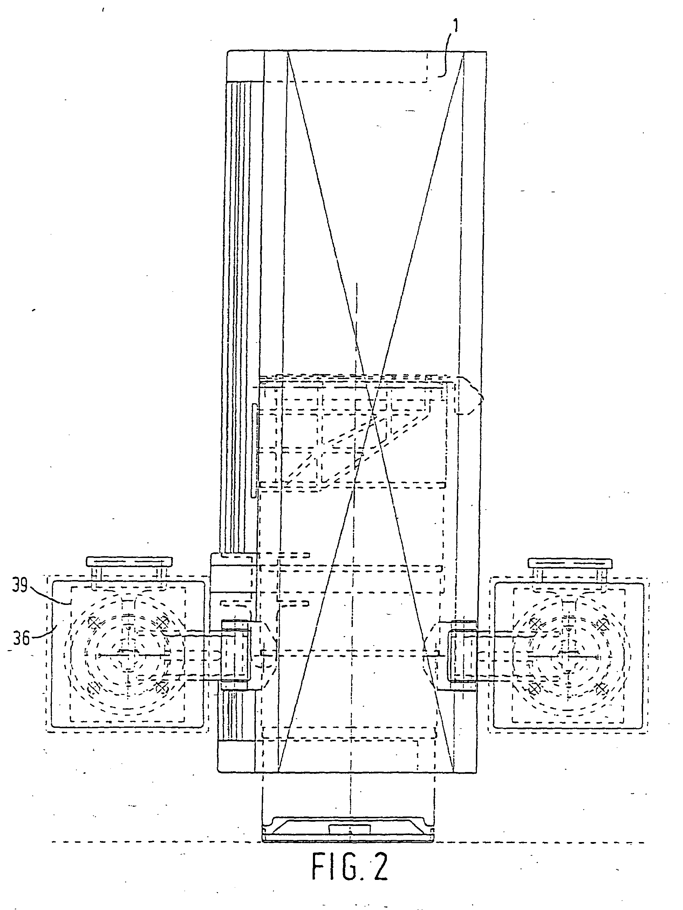

The actual recording surface 39 of image recording element 38, which is illustrated in dotted lines, for example in Figure 2, is rectangular in shape and it is smaller than the outer circumference of the image recording element 36.

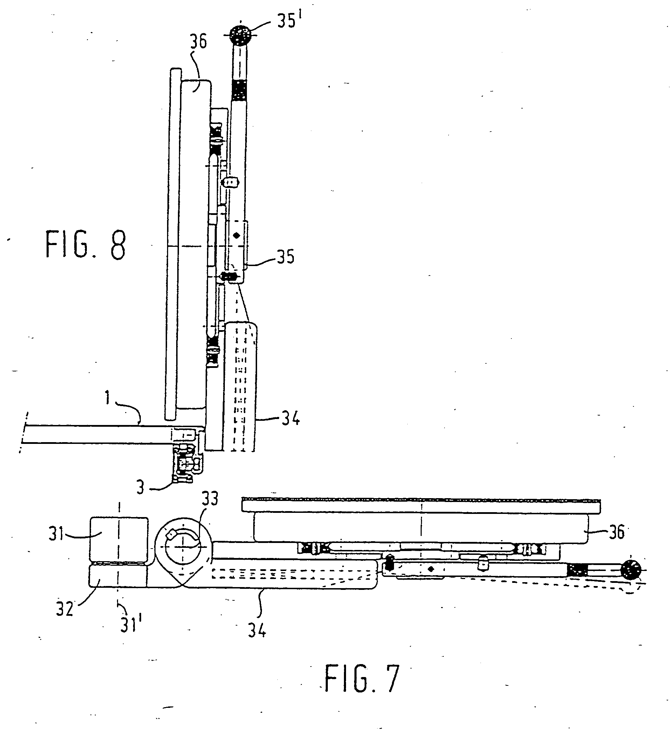

As is shown in Figures 4 and 5, the image recording element can be disposed in several positions 36', 36'' under the tabletop for forming X-ray images of a person who is lying on the tabletop. In addition to that, the image recording element can also pulled out from its position under the table and be disposed in a vertical position, as is shown in Figure 6. By moving the image recording element 36 in vertical direction, the recording surface 39 can be moved to a position in which the lower boundary line of said recording surface 39 is positioned at the same level as the upper side of tabletop 1, as is shown in Figure 6, or in a higher position, in which the entire recording element 36 is disposed above the tabletop, as is indicated in Figure 8.

Furthermore it is possible to dispose the recording element 36 in a horizontal position outside the tabletop, as is shown in Figure 7. In this position of the image recording element it is possible to place parts of the body, for example a person's hands, on the image recording element 36 for making X-ray images.

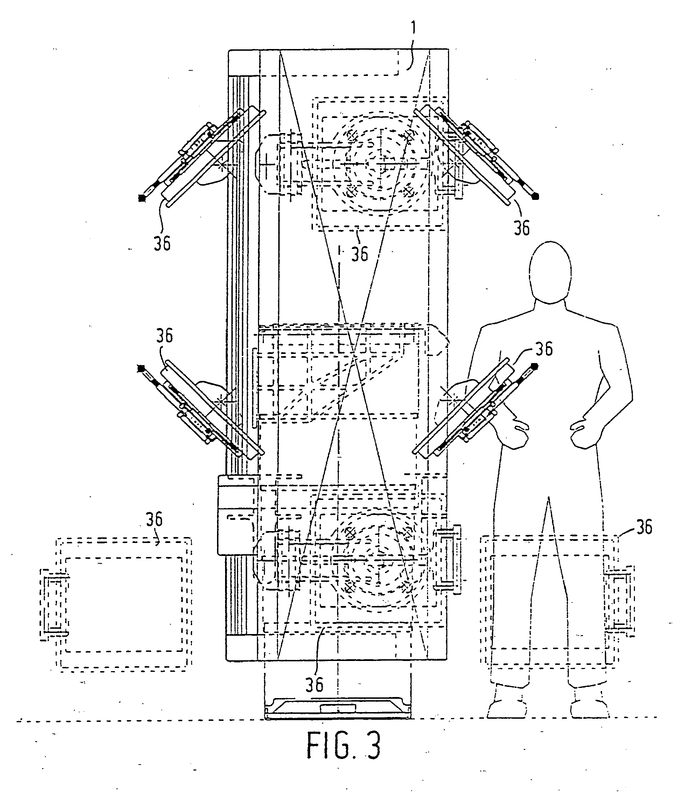

As is apparent from Figures 1 - 3, in which several positions of one and the same image recording element 36 are shown either in dotted lines or in full lines, the image recording element 36 can be moved in vertical direction with respect to tabletop 1 in the vertical position of tabletop 1, whereby the image recording element 36 may be disposed behind or beside the tabletop, as will be apparent from Figures 1 - 3, and the possibilities of pivoting the image recording element 36 about pivots 31' and 33 and the possibility to move the image recording element along support 30 in a direction transversely the longitudinal direction of tabletop 1 and in the longitudinal direction of arm 34 make it possible to position the image recording element 36 optimally with respect to the person to be examined. A further contribution to obtaining an optimum adjustment of the image recording element is obtained by the fact that the image recording element 36 is pivotable with respect to the supporting element 36 that is connected to the arm 34, about a pivot 38 extending perpendicularly to the recording surface 39 of the image recording element, so that also the recording surface 39, which is of rectangular section, can be adjusted to an optimum position. Also the possibility to move the tabletop 1 in two directions extending perpendicularly to each other contributes towards obtaining an optimum adjustment of the image recording element 36 with respect to the person to be examined.

From the foregoing it will be apparent that the device according to the invention makes it possible, using an image recording element 35, to obtain an X-ray image of each part of the body of a person to be examined with an optimum position of the person to be examined and the image recording element with respect to each other.

## Claims

1. A table for X-ray examination, comprising a tabletop (1) supported by a frame, on which a person to be examined can lie in a horizontal position of the tabletop, whilst an image recording element (36) is connected to the tabletop (1), which image recording element (36) is movable in a direction parallel to the longitudinal direction of the tabletop and adjustable between a position in which the image recording element (36) extends perpendicular to the imaginary plane comprising the upperside of the tabletop and a position in which the image recording element extends parallel to said imaginary plane, **characterized in that** the image recording element (6) is supported by an arm (34) which is pivotably about a pivot (33) that extends parallel to the tabletop, along which arm (34) the image recording element (36) is movable and adjustable in several positions.

2. A table according to claim 1, **characterized in that** the image recording element (36) is connected to a slide (31) which is movable along a support (30) that extends under the tabletop transversely to the longitudinal axis thereof to move the image recording element (36) from one side of the tabletop to the other, which support (30) is movable in the longitudinal direction of the tabletop.

3. A table according to claim 2, **characterized in that** the image recording element (36) is pivotable with respect to said slide (31) about a pivot (31') which extends at least substantially perpendicular to the tabletop.

4. Tabletop according to claim 2 or 3, **characterized in that** the image recording element (36) is pivotable with respect to said slide (31) about the pivot (33) extending parallel to the tabletop.

5. A tabletop according to one of the preceding claims, **characterized in that** the image recording element (36) is pivotable with respect to said arm (34, 35) about a pivot (38) extending perpendicular to the recording surface (39) of the image recording element (36).

6. A table according to one of the preceding claims, **characterized in that** the tabletop is vertically adjustable with respect to said frame and pivotably through an angle of ± 90° about a pivot pin (8) extending transversely to the longitudinal axis of the tabletop.

7. A table according to any one of the preceding claims, **characterized in that** the tabletop is adjustable in its longitudinal direction with respect to the frame.

8. A table according to any one of the preceding claims, **characterized in that** the tabletop is adjustable in a direction transversely to its longitudinal direction.

## Patentansprüche

1. Tisch für die Röntgenstrahl-Untersuchung, der eine Tischplatte (1) umfasst, die von einem Rahmen unterstützt ist und auf der eine zu untersuchende Person in einer horizontalen Lage liegen kann, während ein Bildaufzeichnungselement (36) mit der Tischplatte (1) verbunden ist, wobei das Bildaufzeichnungselement (36) in einer Richtung parallel zur Längsrichtung der Tischplatte beweglich ist und einstellbar ist zwischen einer Position, in der sich das Bildaufzeichnungselement (36) senkrecht zu der die Oberseite der Tischplatte enthaltenden Abbildungsebene erstreckt, und einer Position, in der sich das Bildaufzeichnungselement parallel zu der Abbildungsebene erstreckt, **dadurch gekennzeichnet, dass** das Bildaufzeichnungselement (6) durch einen Arm (34) unterstützt ist, der um einen Drehzapfen (33) schwenkbar ist, der sich parallel zur Tischplatte erstreckt, und wobei längs des Arms (34) das Bildaufzeichnungselement (36) in mehrere verschiedene Positionen beweglich und einstellbar ist.

2. Tisch nach Anspruch 1, **dadurch gekennzeichnet, dass** das Bildaufzeichnungselement (36) mit einem Gleiter (31) verbunden ist, der längs eines Trägers (30) beweglich ist, der sich unter der Tischplatte quer zu deren Längsachse erstreckt, um das Bildaufzeichnungselement (36) von einer Seite der Tischplatte zur anderen zu bewegen, wobei der Träger (30) in Längsrichtung der Tischplatte beweglich ist.

3. Tisch nach Anspruch 2, **dadurch gekennzeichnet, dass** das Bildaufzeichnungselement (36) in Bezug auf den Gleiter (31) um einen Drehzapfen (31'), der sich wenigstens im Wesentlichen senkrecht zu der Tischplatte erstreckt, schwenkbar ist.

4. Tischplatte nach Anspruch 2 oder 3, **dadurch gekennzeichnet, dass** das Bildaufzeichnungselement (36) in Bezug auf den Gleiter (31) um den Drehzapfen (33), der sich parallel zu der Tischplatte erstreckt, schwenkbar ist.

5. Tischplatte nach einem der vorhergehenden Ansprüche, **dadurch gekennzeichnet, dass** das Bildaufzeichnungselement (36) in Bezug auf den Arm (34, 35) um einen Drehzapfen (38), der sich senkrecht zu der Aufzeichnungsoberfläche (39) des Bildaufzeichnungselements (36) erstreckt, schwenkbar ist.

6. Tisch nach einem der vorhergehenden Ansprüche, **dadurch gekennzeichnet, dass** die Tischplatte in Bezug auf den Rahmen vertikal einstellbar ist und um einen Winkel von ±90° um einen Schwenkstift (8), der sich quer zu der Längsachse der Tischplatte erstreckt, schwenkbar ist.

7. Tisch nach einem der vorhergehenden Ansprüche, **dadurch gekennzeichnet, dass** die Tischplatte in ihrer Längsrichtung in Bezug auf den Rahmen einstellbar ist.

8. Tisch nach einem der vorhergehenden Ansprüche, **dadurch gekennzeichnet, dass** die Tischplatte in einer Richtung quer zu ihrer Längsrichtung einstellbar ist.

## Revendications

1. Table pour examen aux rayons X, comportant un plateau de table (1) supporté par un bâti, sur laquelle une personne à examiner peut s'étendre dans la position horizontale du plateau de table (1) tandis qu'un élément d'enregistrement d'image (36) est connecté au plateau de table (1), lequel élément d'enregistrement d'image (36) peut être déplacé dans une direction parallèle à la direction longitudinale du plateau de table et être réglé entre une position dans laquelle l'élément d'enregistrement d'image (36) s'étend perpendiculairement au plan imaginaire comprenant le côté supérieur du plateau de table et une position dans laquelle l'élément d'enregistrement d'image s'étend parallèlement audit plan imaginaire, **caractérisé en ce que** l'élément d'enregistrement d'image (36) est supporté par un bras (34) qui peut pivoter autour d'un pivot (33) qui s'étend parallèlement au plateau de table, bras (34) le long duquel l'élément d'enregistrement d'image (36) peut être déplacé et réglé dans plusieurs positions.

2. Table selon la revendication 1, **caractérisée en ce que** l'élément d'enregistrement d'image (36) est connecté à une glissière (31) qui peut se déplacer le long d'un support (30) qui s'étend sous le plateau de table transversalement à son axe longitudinal pour déplacer l'élément d'enregistrement d'image (36) d'un côté du plateau table à l'autre, lequel support (30) peut être déplacé dans la direction longitudinale du plateau de table.

3. Table selon la revendication 2, **caractérisée en ce que** l'élément d'enregistrement d'image (36) peut pivoter par rapport à ladite glissière (31) autour d'un pivot (31') qui s'étend au moins de façon essentiellement perpendiculaire au plateau de table.

4. Plateau de table selon la revendication 2 ou 3, **caractérisé en ce que** l'élément d'enregistrement d'image (36) peut pivoter par rapport à ladite glissière (31) autour du pivot (33) s'étendant parallèlement au plateau de table.

5. Plateau de table selon l'une quelconque des revendications précédentes, **caractérisé en ce que** l'élément d'enregistrement d'image (36) peut pivoter par rapport au dit bras (34, 35) autour d'un pivot (38) s'étendant perpendiculairement à la surface d'enregistrement (39) de l'élément d'enregistrement d'image (36).

6. Table selon l'une quelconque des revendications précédentes, **caractérisée en ce que** le plateau de table peut être réglé verticalement par rapport audit bâti et de façon à pouvoir pivoter sur un angle de ± 90° autour d'une broche de pivotement (8) s'étendant transversalement à l'axe longitudinal du plateau de table.

7. Table selon l'une quelconque des revendications précédentes, **caractérisée en ce que** le plateau de table peut être réglé dans sa direction longitudinale par rapport au bâti.

8. Table selon l'une quelconque des revendications précédentes, **caractérisée en ce que** le plateau de table peut être réglé dans une direction transverse à sa direction longitudinale.
